# EUROPÄISCHE PATENTANMELDUNG

(11) **EP 1 982 583 A2**
(43) Veröffentlichungstag der Anmeldung: **22.10.2008**
(21) Anmeldenummer: 08450053.7
(22) Anmeldetag: 11.04.2008
(51) Int. Cl.: A01K 1/00, A61L 9/14, B05B 7/24

(54) **Verfahren zum Austragen flüssiger Wirkstoffe zur Desodorierung und Desinfektion eines Raumes, insbesondere eines Stalles**

(30) Priorität: 17.04.2007 AT 5882007
(71) Anmelder: Buchrucker, Karl, Sr., 4100 Ottensheim (AT)
(72) Erfinder: Buchrucker, Karl, Sr., 4100 Ottensheim (AT)
(74) Vertreter: Hübscher, Helmut

(57) **Zusammenfassung**

Es wird ein Verfahren zum Austragen flüssiger Wirkstoffe zur Desodorierung und Desinfektion eines Raumes (1), insbesondere eines Stalles, beschrieben, wobei die Wirkstoffe mit Hilfe eines Druckgases durch im Raum (1) verteilte Zerstäuberdüsen (2) zerstäubt werden. Um vorteilhafte Austragungsbedingungen zu schaffen, wird vorgeschlagen, dass die Wirkstoffe in den Zerstäubungsdüsen (2) wahlweise durch ein Feinzerstäuben mit Hilfe eines höheren Gasdruckes zu einem Aerosol oder durch ein Grobzerstäuben mit Hilfe eines niedrigeren Gasdruckes zu einem absinkenden Flüssigkeitsnebel zerstäubt werden.

## Beschreibung

Die Erfindung bezieht sich auf ein Verfahren zum Austragen flüssiger Wirkstoffe zur Desodorierung und Desinfektion eines Raumes, insbesondere eines Stalles, wobei die Wirkstoffe mit Hilfe eines Druckgases durch im Raum verteilte Zerstäuberdüsen zerstäubt werden.

Es sind Wirkstoffe bekannt, die sich sowohl zur Desodorierung als auch zur Desinfektion eignen und beispielsweise eine Kombination aus mehrwertigen Alkoholen, Lösungsvermittlern und ätherischen Ölen enthalten. Diese flüssigen Wirkstoffe sind mit Wasser unbeschränkt verdünnbar und können sowohl durch ein Zerstäuben als auch durch ein Verdampfen ausgebracht werden. Zum Zerstäuben, das sich in der praktischen Anwendung besonders bewährt hat, wird der flüssige Wirkstoff in einer entsprechenden Verdünnung mit Hilfe eines Druckgases, üblicherweise Luft, in Zerstäuberdüsen fein zerstäubt, wobei die Bildung von Aerosolen angestrebt wird, um den Wirkstoff möglichst lange in der Raumluft zu halten und damit eine besonders wirkungsvolle Anwendung insbesondere im Bereich von Räumen zu ermöglichen, die die Gefahr einer Geruchsbelastung der Umwelt mit sich bringen, wie dies beispielsweise bei einer Intensivtierhaltung der Fall ist.

Zur Erzeugung beständiger Aerosole ist es in diesem Zusammenhang bekannt (WO 2006/039918 A2), den flüssigen Wirkstoff unter Druck zu einer Zerstäuberdüse zu fördern, die den Wirkstoff in einen durch den Düsenstrahl injizierten Luftstrom zerstäubt. Dieses Luftwirkstoffgemisch wird in wenigstens einer nachgeordneten mit Druckluft beaufschlagten Mischkammer verdünnt, wobei mit Hilfe eines Druckreglers der Beaufschlagungsdruck der Mischkammer so eingestellt werden kann, dass sich eine gleichmäßige, sehr feine Verteilung des zerstäubten Wirkstoffes im aus der Mischkammer austretenden Luftstrom ergibt. Es hat sich allerdings herausgestellt, dass trotz der Erzeugung sehr beständiger Aerosole aus den eingesetzten Wirkstoffen die Desinfektionswirkung dieser Aerosole hinter den Erwartungen zurückblieb, vor allem bei erhöhten Belastungen durch Krankheitskeime und drohender Infektionsgefahr.

Der Erfindung liegt somit die Aufgabe zugrunde, ein Verfahren zur Ausbringung flüssiger Wirkstoffe zur Deodorierung und Desinfektion von Räumen, insbesondere Ställen, der eingangs geschilderten Art so auszugestalten, dass sowohl die deodorierenden als auch die keimreduzierenden Eigenschaften der Wirkstoffe vorteilhaft genützt werden können.

Die Erfindung löst die gestellte Aufgabe dadurch, dass die Wirkstoffe in den Zerstäubungsdüsen wahlweise durch ein Feinzerstäuben mit Hilfe eines höheren Gasdruckes zu einem Aerosol oder durch ein Grobzerstäuben mit Hilfe eines niedrigeren Gasdruckes zu einem absinkenden Flüssigkeitsnebel zerstäubt werden.

Der Erfindung liegt die Erkenntnis zugrunde, dass nur dann eine wirkungsvolle Desinfektion bei einer erhöhten Infektionsgefahr bzw. bei höheren Belastungen durch Krankheitskeime erreicht werden kann, wenn für eine Benetzung der Bodenflächen und der sich im Raum befindlichen Gegenstände mit dem Wirkstoff gesorgt werden kann. Da die Einbringung des Wirkstoffes in den zu desinfizierenden Raum in Aerosolform allein hiefür nicht ausreicht, wird der Wirkstoff erfindungsgemäß mit Hilfe eines niedrigeren Gasdruckes zu einem Flüssigkeitsnebel mit ausreichend großen Flüssigkeitströpfchen zerstäubt, um schwerkraftbedingt gegen den Boden abzusinken, sodass durch den sich absenkenden Flüssigkeitsnebel eine Benetzung der sich im Bereich dieses Flüssigkeitsnebels befindlichen Flächen sichergestellt wird. Für die desodorierende Wirkung ist die Verteilung der Wirkstoffe im Raum als Aerosol anzustreben, was eine Feinzerstäubung der Wirkstoffe unter einem höheren Gasdruck erfordert. Besonders günstige Verhältnisse ergeben sich in diesem Zusammenhang, wenn die Feinzerstäubung und die Grobzerstäubung der Wirkstoffe zeitlich verteilt abwechselnd durchgeführt wird, um vorteilhafte Bedingungen sowohl hinsichtlich der Desodorierung als auch der Desinfektion gewährleisten zu können. Als vorteilhafter zusätzlicher Effekt hat sich herausgestellt, dass durch die Benetzung von allfälligen Feinstoffanteilen in der Raumluft mit den Aerosolen eine Feinstaubausfällung erreicht werden kann, wobei sich durch die Wirkstoffe eine Bindung ergibt, die ein neuerliches Aufwirbeln der abgeschiedenen Feinstoffanteile verhindert.

Zur Durchführung eines solchen Verfahrens zum Austragen flüssiger Wirkstoffe zur Desodorierung und Desinfektion eines Raumes kann von in diesem Raum verteilten Zerstäuberdüsen ausgegangen werden, die einerseits an eine Druckgasleitung und anderseits an eine Wirkstoffleitung angeschlossen sind. Zur wahlweisen Fein- oder Grobzerstäubung der Wirkstoffe in den Zerstäuberdüsen braucht lediglich die Druckgasleitung mit einem höheren oder niedrigeren Gasdruck beaufschlagt zu werden. Damit nicht unterschiedliche Druckgasquellen bereitgestellt werden müssen, kann die Druckgasleitung wahlweise über einen von zwei parallel geschalteten Druckreglern einerseits für einen höheren und andererseits für einen niedrigeren Gasdruck an einer gemeinsamen Druckgasquelle angeschlossen werden, sodass sich ein vergleichsweise geringer Konstruktionsaufwand ergibt.

Kann die Wirkstoffleitung wahlweise mit einem Wirkstoffbehälter oder einer Wasserleitung verbunden werden, so kann die Vorrichtung zum Austragen der flüssigen Wirkstoffe zusätzlich zur Steuerung des Raumklimas eingesetzt werden, weil durch einen fein zerstäubten Wassernebel nicht nur die Luftfeuchtigkeit im Raum gesteigert, sondern auch die Raumluft gekühlt werden kann, wenn der Raumluft die für das Verdampfen des Wassernebels erforderliche Verdampfungswärme entzogen wird. Da über den Beaufschlagungsdruck der Gasdruckleitung Einfluss auf die Tröpfchengröße des zerstäubten Wassers genommen werden kann, kann auch für die Benetzung der Bodenflächen des Raumes gesorgt werden, um beispielsweise die Reinigung des Raumes zu erleichtern.

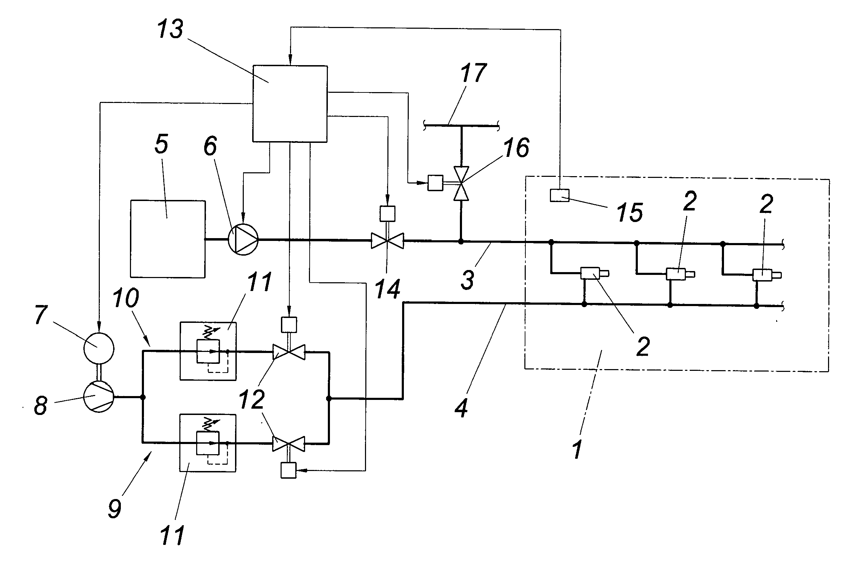

Anhand der Zeichnung wird das erfindungsgemäße Verfahren näher erläutert, und zwar wird eine erfindungsgemäße Vorrichtung zum Austragen flüssiger Wirkstoffe zur Desodorierung und Desinfektion eines Raumes in einem vereinfachten Blockschaltbild gezeigt.

Um in einem strichpunktiert angedeuteten Raum 1, beispielsweise in einem Stall, flüssige Wirkstoffe zur Desodorierung und zur Desinfektion auszubringen, sind im Raum 1 Zerstäuberdüsen 2 vorgesehen, die einerseits an eine Wirkstoffleitung 3 und anderseits an eine Druckgasleitung 4 angeschlossen sind. Über die Wirkstoffleitung 3 werden die flüssigen Wirkstoffe aus einem Vorratsbehälter 5 mittels einer Pumpe 6 den Zerstäuberdüsen 2 zugefördert. Die Versorgung der Zerstäuberdüsen 2 mit einem Druckgas, vorzugsweise mit Druckluft, erfolgt über einen von einem Motor 7 angetriebenen Verdichter 8, dessen Druckleitung sich in zwei in die Druckgasleitung 4 mündende Äste 9 und 10 verzweigt, die jeweils mit einem Druckregler 11 und einem Schaltventil 12 versehen sind. Während der Ast 9 einen den Gasdruck auf ein niedriges Druckniveau von beispielsweise 0,5 bis 1,5 bar reduzierenden Druckregler 11 aufweist, sorgt der Druckregler 11 im Ast 10 für ein höheres Druckniveau, das beispielsweise zwischen 2 und 5 bar eingestellt werden kann. Die Einstellung des Gasdruckes durch die Druckregler 11 wird im Zusammenwirken mit den Zerstäuberdüsen 2 so gewählt, dass bei einer Beaufschlagung der Druckgasleitung 4 mit dem höheren Druck die über die Wirkstoffleitung 3 angeförderten Wirkstoffe zu einem Aerosol fein zerstäubt werden, bei der Beaufschlagung der Druckgasleitung 4 mit einem niedrigeren Gasdruck jedoch ein Wirkstoffnebel mit gröberen Wassertröpfchen entsteht, die zumindest mehrheitlich schwerkraftbedingt absinken. Durch diese unterschiedliche Ausbringung der Wirkstoffe in den Raum 1 wird den Anforderungen sowohl einer Desodorierung als auch einer Desinfektion und einer Feinstaubbindung vorteilhaft entsprochen, insbesondere wenn die Wirkstoffe abwechselnd in zeitlichen Abständen einer Fein- und einer Grobzerstäubung unterworfen werden.

Wie dem Blockschaltbild entnommen werden kann, wird die Ausbringung der Wirkstoffe in den Raum 1 mit Hilfe einer Steuereinrichtung 13 automatisiert, die in vorgebbaren zeitlichen Abständen den Stelltrieb eines Schaltventils 14 in der Wirkstoffleitung 3 zusammen mit dem Stelltrieb eines der beiden Schaltventile 12 in den Ästen 9 und 10 der Druckleitung des Verdichters 8 ansteuert, um die Wirkstoffe in zeitlichen Intervallen von beispielsweise 15 Minuten abwechselnd unter einer Feinzerstäubung z. B. für 10 Sekunden und einer Grobzerstäubung z. B. für 15 Sekunden in den Raum 1 auszutragen. Da die Austragung der Wirkstoffe in zeitlichen Intervallen erfolgt, werden der Motor 7 für den Verdichter 8 und die Pumpe 6 für die Wirkstoffe über die Steuereinrichtung 13 nur für die Zerstäubungsdauer der Wirkstoffe eingeschaltet. Damit die angestrebte Wirkung der zerstäubten Wirkstoffe im erwarteten Umfang tatsächlich eintritt, müssen die Raumtemperatur und die Luftfeuchtigkeit im Raum 1 berücksichtigt werden. Aus diesem Grunde werden diese Parameter mit Hilfe eines Fühlers 15 erfasst und an die Steuereinrichtung 13 weitergeleitet, sodass beim Unterschreiten vorgegebener Grenzwerte für die Raumtemperatur bzw. beim Überschreiten entsprechender Grenzwerte für die Luftfeuchtigkeit die Wirkstoffeinbringung in den Raum 1 ausgesetzt werden kann.

Da an die Wirkstoffleitung 3 über ein von der Steuereinrichtung 13 ansteuerbares Schaltventil 16 eine Wasserleitung 17 angeschlossen werden kann, wird es mit Hilfe der dargestellten Vorrichtung möglich, nicht nur Wirkstoffe, sondern auch Wasser in unterschiedlicher Zerstäubungsform im Raum 1 zu verteilen, was eine zusätzliche Möglichkeit zur Einflussnahme auf das Raumklima eröffnet, weil mit Hilfe des fein zerstäubten Wassers die Luftfeuchtigkeit angehoben und die Temperatur abgesenkt werden kann. Bei einer Grobzerstäubung kann dabei die Benetzung von Bodenflächen sichergestellt werden, was eine Reinigung dieser Bodenflächen erleichtern kann.

## Patentansprüche

1. Verfahren zum Austragen flüssiger Wirkstoffe zur Desodorierung und Desinfektion eines Raumes (1), insbesondere eines Stalles, wobei die Wirkstoffe mit Hilfe eines Druckgases durch im Raum (1) verteilte Zerstäuberdüsen (2) zerstäubt werden, **dadurch gekennzeichnet, dass** die Wirkstoffe in den Zerstäubungsdüsen (2) wahlweise durch ein Feinzerstäuben mit Hilfe eines höheren Gasdruckes zu einem Aerosol oder durch ein Grobzerstäuben mit Hilfe eines niedrigeren Gasdruckes zu einem absinkenden Flüssigkeitsnebel zerstäubt werden.

2. Verfahren nach Anspruch 1, **dadurch gekennzeichnet, dass** die Wirkstoffe in zeitlichen Abständen abwechselnd einer Fein- und Grobzerstäubung unterworfen werden.

3. Vorrichtung zur Durchführung eines Verfahrens zum Austragen flüssiger Wirkstoffe zur Desodorierung und Desinfektion eines Raumes (1), insbesondere eines Stalles, nach Anspruch 1 oder 2 mit im Raum (1) verteilten, einerseits an eine Druckgasleitung (4) und anderseits an eine Wirkstoffleitung (3) angeschlossenen Zerstäuberdüsen (2), **dadurch gekennzeichnet, dass** die Druckgasleitung (4) mit einem höheren oder niedrigeren Gasdruck zur wahlweisen Fein- oder Grobzerstäubung des Wirkstoffes beaufschlagbar ist.

4. Vorrichtung nach Anspruch 2, **dadurch gekennzeichnet, dass** die Druckgasleitung (4) wahlweise über einen von zwei parallel geschalteten Druckreglern (11) einerseits für einen höheren und anderseits für einen niedrigeren Gasdruck an eine gemeinsame Druckgasquelle (8) angeschlossen ist.

5. Vorrichtung nach Anspruch 2 oder 3, **dadurch gekennzeichnet, dass** die Wirkstoffleitung (3) wahlweise mit einem Wirkstoffbehälter (5) oder einer Wasserleitung (17) verbindbar ist.
